# FASCICULE DE BREVET EUROPEEN

(11) **EP 2 862 529 B1**
(45) Date de publication et mention de la délivrance du brevet: **06.04.2016**
(21) Numéro de dépôt: 14003529.6
(22) Date de dépôt: 15.10.2014
(51) Int. Cl.: A61B 17/70

(54) **Dispositif et système de fixation vertébrale et méthode de blocage d'une boucle avec un tel dispositif**
Wirbelbefestigungsvorrichtung und -system und Methode zum Blockieren einer Öse mit einer solchen Vorrichtung
Vertebral fixation device and system and method for blocking a loop with such a device

(30) Priorité: 18.10.2013 FR 1360195
(43) Date de publication de la demande: 22.04.2015
(73) Titulaire: Implanet, Société Anonyme, 33650 Martillac (FR)
(72) Inventeur: Le Couëdic, Régis, 33000 Bordeaux (FR); Baccelli, Christian, 33650 Saucats (FR)
(74) Mandataire: Benech, Frédéric

(56) Documents cités:
- WO-A1-2013/001180
- FR-A1- 2 954 905

## Description

La présente invention concerne un dispositif de fixation vertébrale pour maintien d'une vertèbre rachidienne sur une tige, du type comportant un élément de fixation sur la tige, comprenant une pièce de section transversale en forme de U ou sensiblement en forme de U présentant une première branche, un fond et une seconde branche, comprenant une boucle formée par le rapprochement de deux portions d'extrémité d'une bande souple de liaison de la vertèbre avec la pièce, la pièce étant muni sur chacune de ses branches d'un évidement en vis à vis de passage des portions d'extrémité de la bande, et comportant des moyens de blocage réglables des deux portions d'extrémité de la bande souple sur la paroi du fond du U par la tige.

Elle concerne également un système comprenant un tel dispositif et sa tige attenante.

Elle vise également une méthode de blocage d'une boucle fermée par rapport à une tige.

Elle trouve une application particulièrement importante bien que non exclusive dans le domaine du redressement de la colonne vertébrale d'un patient présentant une courbure anormale.

Afin de redresser l'ensemble il est connu de rapprocher les bords latéraux des vertèbres de part et d'autre de la colonne vertébrale, par le biais de tiges reliant entre elles, soit des vis, que l'on insère dans les vertèbres elles-mêmes, soit des crochets, que l'on introduit le long du canal rachidien.

Ces dispositifs ne sont néanmoins pas satisfaisants.

L'utilisation de vis implique que les vertèbres soient en bon état.

L'utilisation de crochets est quant à elle délicate et comporte un risque d'accident important pouvant entraîner la paralysie du patient.

Pour pallier ces inconvénients il a été proposé (FR 2 954 905) un système qui évite les vis de fixation ou les crochets.

Le système comprend un lien flexible de fixation de la vertèbre sur une pièce de liaison, elle-même fixée à la tige.

Des moyens de blocage des extrémités ou portions d'extrémités de la boucle passée autour de la vertèbre par coincement sur la tige d'une vis partiellement conique sont prévus.

Si un tel système permet d'obtenir un serrage progressif efficace, il peut entrainer dans certains cas, notamment lorsque la tige est en matière non métallique, un relâchement de la tension de la bande souple avec le temps.

La présente invention vise à pallier les inconvénients de l'art antérieur.

Pour ce faire, elle vise à fournir un dispositif de fixation vertébrale répondant mieux que ceux antérieurement connus aux exigences de la pratique, notamment en ce qu'elle va permettre une meilleure tenue de la tension de la bande souple pendant la vie de la prothèse, et donc de maintien de la tige en position par rapport aux vertèbres, en ce qu'elle autorise une meilleure souplesse et une plus grande flexibilité de montage des portions d'extrémités de la boucle dans la pièce de fixation du dispositif sur la tige et en ce qu'elle présente des possibilités de réglage plus diversifiées du fait d'un montage modulaire, et ce en fonction des matériaux et des épaisseurs de bande.

Dans ce but, l'invention propose essentiellement un dispositif de fixation vertébrale pour maintien d'une vertèbre rachidienne sur une tige, comportant un élément de fixation comprenant une pièce de section transversale en forme de U ou sensiblement en forme de U présentant une première branche, un fond et une seconde branche, lesdites branches comportant une portion distale, une boucle formée par le rapprochement de deux portions d'extrémité d'une bande souple de liaison de la vertèbre avec la pièce, ladite pièce étant munie sur chacune de ses branches d'un évidement de passage des portions d'extrémité de la bande, lesdits évidements étant situés en vis à vis l'un de l'autre, et des moyens de blocage réglables des deux portions d'extrémité de la bande souple sur la paroi du fond du U par la tige enfilée sur la pièce, comprenant une vis de liaison comportant un corps reliant les portions distales en vis à vis des deux branches du U, ledit corps étant muni d'un côté d'une tête de vissage et de blocage longitudinal de la vis par rapport à une première branche du U et de l'autre côté d'un pas de vis de vissage dans la seconde branche du U, caractérisé en ce que les moyens de blocage comportent de plus un manchon amovible de passage de la vis dans la première branche du U, en butée longitudinale avec la tête de la vis et comportant une partie chanfreinée agencée pour coopérer avec la tige le long d'une génératrice de cette dernière et pour comprimer les portions d'extrémité de la bande entre la tige et le fond du U lors du serrage.

Avantageusement la partie chanfreinée est plane et forme un pan coupé.

Mais également avantageusement la partie chanfreinée et bombée (concave ou convexe), allongée autour d'un axe parallèle (ou sensiblement parallèle) à celui de la tige enfilée sur la pièce.

Le fait que la partie du manchon comprenne une partie chanfreinée par exemple formant pan coupé, agissant sur une ligne génératrice de la tige, va autoriser un serrage homogène tout au long de cette ligne génératrice, qui est donc en contact avec le chanfrein sur une longueur déterminée et donc non ponctuellement.

De ce fait, on observe que le blocage de la bande a une plus grande pérennité dans le temps, et d'autant plus qu'un écrasement de la tige va également pouvoir se faire de façon plus répartie, sans effet de poinçonnement.

Dans des modes de réalisation avantageux, on a de plus et/ou par ailleurs recours à l'une et/ou à l'autre des dispositions suivantes :
- la pièce est formée d'un seul tenant ;
- le pan coupé forme un angle compris entre 30° et 80° avec le plan perpendiculaire à l'axe de la vis, par exemple 60°;
- la paroi interne du fond du U est en forme de portion de cylindre, terminée d'un côté par un rebord dans le sens parallèle à la tige autorisant un blocage de la tige dans le fond du U ;

- la tête de la vis de serrage comporte un épaulement propre à coopérer avec la face externe de la partie supérieure du manchon ;
- la vis et la pièce étant en titane, le manchon et la tige sont en matériau polymère, et la bande souple est une tresse en polymère ;
- la vis est en titane, le manchon, la pièce et la tige étant en matériau polymère et la bande souple est une tresse en polymère ;
- la vis, la pièce de liaison et la tige sont en titane, le manchon étant en polymère, et la bande souple est une tresse en polymère ;
- le manchon comprend une partie en portion de cylindre d'un côté et se termine de l'autre côté, situé vers la tige, par une partie allongée plus étroite que le diamètre de la partie en portion de cylindre comportant la partie chanfreinée, la première branche du U comportant un orifice de passage du manchon au moins en partie de forme complémentaire à celle de la partie supérieure dudit manchon et s'étendant vers le fond du U pour former l'évidement de passage des portions d'extrémité de la bande pour la première branche du U ;
- la partie allongée plus étroite est biseautée, symétrique par rapport au plan central transversal du manchon et présente deux côtés opposés plans formant entre eux un angle α. Avantageusement l'angle α est compris entre 15° et 45°, par exemple 30°.
- Il a été en effet observé que la tige est rarement rectiligne dans le plan orthogonal à l'axe de la vis. L'utilisation du contact ponctuel est donc plutôt recherchée par l'homme du métier, car un implant ayant une zone de contact plus large peut être difficile à assembler. En réduisant la taille de la génératrice de contact grâce à une forme biseautée selon α, on obtient un bon compromis ;
- l'orifice de passage de la première branche du U s'étend vers le fond pour former l'évidement de passage de la bande en présentant deux côtés latéraux symétriques par rapport à l'axe de la pièce en formant entre eux un angle γ < α. Le fait que cet angle γ soit inférieur ou légèrement inférieur (de quelques degrés) à l'angle α permet une adaptabilité du système. Le manchon peut ainsi se positionner librement en fonction de l'état de la zone qu'il rencontre en arrivant au contact avec la tige. Il en résulte qu'une fois le contact obtenu entre la tige et le pan coupé, la bissectrice de l'angle α n'est pas forcément contenue par le plan transversal du manchon. L'évidement est donc ainsi conçu et dimensionné pour permettre une légère liberté angulaire du manchon de part et d'autre du plan transversal ;
- le manchon et la première branche du U comportent des moyens d'indexage de la position du manchon dans ladite première branche.

L'invention concerne également un système de redressement de colonne vertébrale mettant en oeuvre un dispositif tel que décrit ci-dessus.

Elle concerne aussi un système de redressement d'une colonne vertébrale comprenant au moins deux dispositifs tels que décrits ci-dessus et au moins une tige cylindrique sur laquelle sont fixés les dispositifs.

L'invention concerne également une méthode de blocage d'une boucle formée par les deux extrémités d'une bande souple sur une tige à l'aide d'une pièce de fixation de section transversale en forme de U, caractérisée en ce que on bloque les extrémités entre le fond du U et la tige par vissage et blocage longitudinal d'une vis au travers d'un manchon amovible du passage de la vis, ledit manchon étant en butée d'un côté avec l'extrémité de la branche du U via la tête de vis et de l'autre côté avec la tige via une partie chanfreinée.

Avantageusement, le manchon pouvant se positionner librement dans un évidement de passage dans une branche du U, on ajuste celui-ci en position avec la tige en fonction de la zone qu'il rencontre en arrivant au contact avec la tige.

Egalement avantageusement la vis et le manchon sont préassemblés de façon à être solidaires en ne conservant qu'une liberté de rotation de l'un par rapport à l'autre.

L'invention sera mieux comprise à la lecture de la description qui suit d'un mode de réalisation donné ci-après à titre d'exemple non limitatif. La description se réfère aux dessins qui l'accompagnent dans lesquels :
La figure 1 est une vue en coupe axiale et partielle du dispositif avec tige cylindrique, selon un mode de réalisation de l'invention.
La figure 2 est une vue de dessus du dispositif de la figure 1.
La figure 3 montre, en vue de dessus, le dispositif de la figure 1 fixant une vertèbre à une tige.
La figure 4 est une vue en perspective du système mettant en oeuvre le dispositif de la figure 1.
La figure 5 est une vue en perspective en pièces détachées, du système de la figure 4.

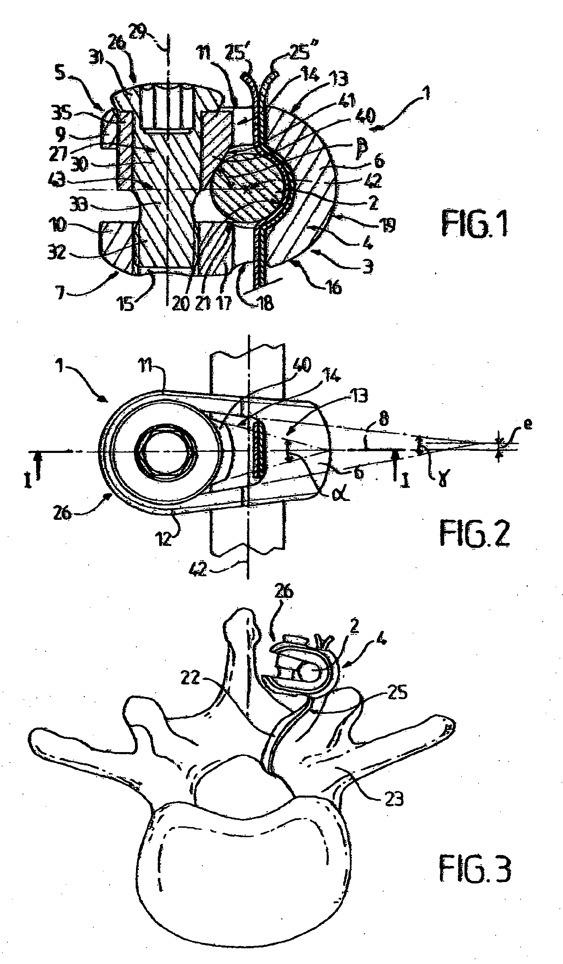

Les figures 1 et 2 montrent un dispositif 1 de fixation vertébrale, pour maintien d'une vertèbre rachidienne sur une tige cylindrique 2.

Il comporte un élément 3 de fixation comprenant une pièce 4 d'un seul tenant, de section transversale sensiblement en forme de U.

La pièce 4 présente une première branche 5, un fond 6 et une seconde branche 7.

Les première et seconde branches 5, 7 du U sont constituées par deux plaquettes ou languettes ajourées, par exemple de 5 mm d'épaisseur comprenant chacune deux parties latérales symétriques par rapport à un plan transversal 8 (voir figure 2).

Chaque plaquette comporte une portion distale 9, 10 semi cylindrique à l'extrémité de chacune desdites branches, reliée au fond 6, pour la première branche 5, par deux bras 11 et 12 écartés l'un de l'autre et délimitant avec la portion distale 9 d'un côté et le dessus 13 du fond 6, un évidement 14 dans la branche 5, de forme semi cylindrique du côté de la portion distale et terminée vers le fond par une portion sensiblement trapézoïdale dont les côtés du trapèze forment un angle γ, par exemple de 10°, et qui sera également détaillée ci-après.

La deuxième branche 7 est quant à elle formée par la portion distale 10, percée d'un alésage 15, et est reliée au-dessous 16 du fond par une portion rectangulaire 17 terminée par un évidement 18 situé en vis à vis de l'extrémité de l'évidement 14 de la branche 5, située du côté du fond.

Le fond 6 comporte quant à lui d'un côté une paroi externe 19 arrondie, de surface en partie cylindrique (ou en partie torique), et de l'autre côté une paroi interne. 20 homothétique ou parallèle à la paroi externe 19, constituant le fond d'une gorge cylindrique de passage de forme sensiblement complémentaire à celle de la tige 2.

Dans le mode de réalisation plus particulièrement décrit ici la paroi 20 comprend en partie inférieure, un rebord longitudinal 21 dans le sens parallèle à la tige 2, formé par l'arête supérieure de la portion rectangulaire 17, la tige étant bloquée par la pression de la portion en coin du manchon dans une zone qui se trouve dans la zone inférieure du fond du U 6.

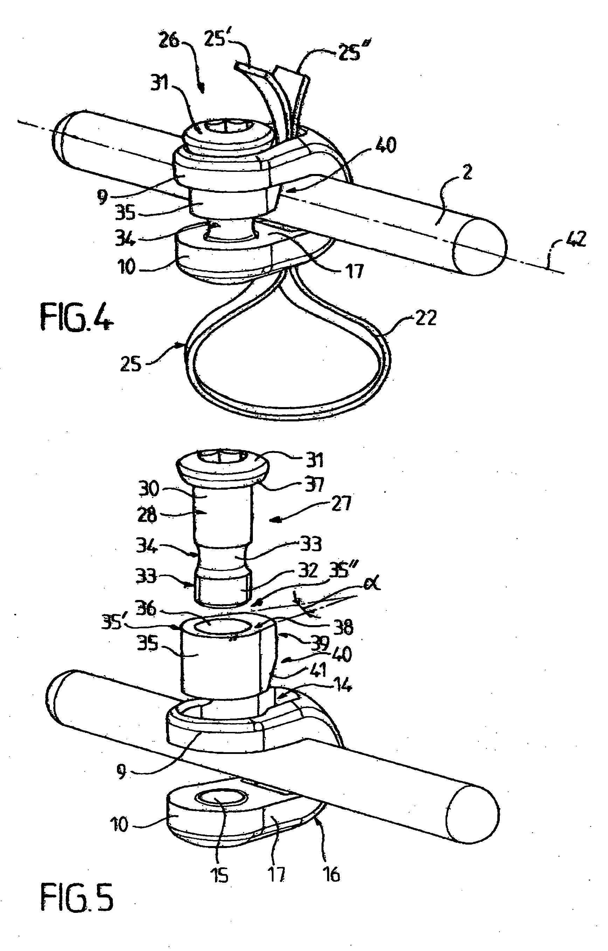

L'élément 3 de fixation comprend de plus une boucle 22 (voir. figures 3 et 4) de fixation sur une vertèbre 23, et plus particulièrement agencé pour être fixée sur la lame de la vertèbre en passant par le canal rachidien 24.

La boucle est formée par une bande 25 souple en polymère tressé, par exemple en polyester de 1 à 3 mm d'épaisseur, de 6 mm de large et de 30 cm de long.

Plus précisément la boucle 22 est formée par le rapprochement des deux portions d'extrémité 25', 25" de la bande 25 souple et assure donc la liaison de la vertèbre 23 avec la pièce 4 de fixation.

A noter que les évidements 14 et 18 sont situés (pour leurs portions situées du côté du fond) en vis-à-vis l'un de l'autre et forment chacun une fente large, par exemple 5 à 10 fois plus large qu'une double épaisseur de bande 25 pour l'évidement 18, et de dimension encore plus grande pour l'évidement 14, et ce pour faciliter son introduction lors de l'opération.

Le dispositif 1 comprend également des moyens 26 de blocage des deux portions d'extrémité 25', 25" de la bande 25 sur la paroi 20 du fond 6 du U, par la tige 2.

Plus précisément en référence aux figures 1 et 5 les moyens 26 de blocage sont réglables et comprennent une vis 27 de liaison comportant un corps 28 agencé pour relier les portions distales 9, 10 en vis-à-vis des deux branches 5 et 7 du U.

Le corps 28 s'étend autour d'un axe longitudinal 29 et comprend une partie supérieure 30 cylindrique terminée par une tête 31 pour le vissage et le blocage longitudinal de la vis par rapport à la première branche 5.

Le corps 28 comporte également une partie inférieure 32 cylindrique muni d'un pas de vissage dans l'alésage fileté 15 de la seconde branche 7 du U.

Dans le mode de réalisation décrit ici, la partie supérieure 30 et la partie inférieure 36 sont reliées entre elles par une partie intermédiaire 33 formant une gorge annulaire 34.

La gorge 34 s'étend par exemple sur une hauteur de la vis 27 comprise entre 1/6^{ème} et 1/3 de celle-ci, et est de faible profondeur, par exemple de 0,5 mm.

Selon le mode de réalisation plus particulièrement décrit ici, les moyens 26 de blocage comportent de plus un manchon 35 amovible de passage de la vis 27.

Le manchon est de forme externe complémentaire à l'évidement 14 du côté de la portion distale 9 et est agencé pour être inséré à frottement doux dans ledit évidement 14.

Il comporte un alésage 36 (cf. figure 5) de passage de la vis 27.

La tête 31 de la vis 27 de serrage comporte quant à elle un épaulement 37 propre à coopérer avec la face externe 38 supérieure du manchon.

Plus précisément le manchon 35 comprend une partie supérieure 39 et une partie inférieure 40 toutes deux d'un côté, en portion de cylindre 35' et de l'autre côté en portion biseautée plus allongée 35", et en forme de secteur torique dans une plage angulaire α comprise entre 20° et 90°, par exemple 30° (cf. figure 2).

La partie inférieure 40 de la portion biseautée 35" du manchon 35 est chanfreinée, parallèlement à l'axe 29 de la vis et de l'alésage pour former un pan coupé 41 du côté du fond du U. Le pan coupé 41 est compris dans un plan parallèle à l'axe 42 de la tige 2 et est agencé pour coopérer avec la tige 2 et pour comprimer les portions d'extrémité 25', 25" de la bande 25 entre la tige 2 et le fond 20 du U lors du serrage.

Il forme un angle β compris entre 30° et 80° avec le plan 43 perpendiculaire à l'axe 29 de la vis, ici de 60°.

La portion plus allongée biseautée du manchon 35 coopère symétriquement à frottement avec les parois en vis à vis de l'évidement 14 de la première branche 5 du U et peut soit former des moyens d'indexage de la position du manchon 35 dans la première branche, soit au contraire, comme dans le cas décrit ici présenter un jeu, du fait de la différence entre les angles α et γ (avec γ < α), ce qui peut entrainer (cf. figure 2) un décalage e entre le plan 8 et le sommet de l'angle γ.

Une telle différence permet un ajustement facilitant la mise en place du dispositif.

On va maintenant décrire, toujours en référence plus particulièrement aux figures 1 et 5, le fonctionnement du dispositif 1.

Il va permettre le blocage en position serrée de la boucle 22 formée par les deux extrémités 25', 25" de la bande 25 souple sur la tige 2 à l'aide de la pièce 4 de fixation, pour autoriser la fixation mécanique de ladite boucle 22 autour de la vertèbre 23.

L'utilisation d'un tel dispositif est particulièrement indiquée dans le cadre d'une chirurgie du rachis du type scoliose, mettant par exemple en oeuvre deux tiges parallèles placées de part et d'autre de la colonne du patient.

Cependant un tel dispositif peut s'avérer également très utile dans des applications dégénératives, en renfort de vis pédiculaires implantées dans un os de mauvaise qualité. Dans d'autres cas de pathologies dégénératives primaires, il peut être envisagé d'utiliser uniquement un tel dispositif en remplacement des vis pédiculaires. Dans cet optique et afin de garder une certaine souplesse à la portion de colonne vertébrale opérée on peut vouloir utiliser des tiges en polymère possédant un module d'élasticité proche de celui de l'os. Dans ce cas, il est nécessaire d'optimiser le contact avec la tige afin d'éviter un fluage pouvant être néfaste à la survie à long terme du système.

Après ouverture de la portion dorsale du patient (non représenté), pour accéder aux vertèbres 23 à redresser, le chirurgien dispose les deux tiges 2.

Il réalise ensuite la mise en place des dispositifs 1 en matériau biocompatible.

On va décrire ici après la mise en place d'un seul dispositif. Il va de soi que la pose des autres dispositifs va être réalisée de façon similaire et progressivement équilibrée.

Dans l'exemple décrit, la vis 27 de fixation et le manchon 35 sont en titane, la tige 2 et la pièce 4 de liaison étant en titane.

Le chirurgien forme tout d'abord la boucle 22 de façon lâche autour de la vertèbre 23 puis passe les portions d'extrémité 25', 25" de la bande 25 dans les évidements 14 et 18 de la pièce 4 et les fait ressortir de l'autre côté par rapport à la boucle.

Il vient ensuite encliquer le dispositif sur la tige 2 qui vient comprimer légèrement les deux portions d'extrémité 25', 25" de la bande contre le fond du U, ce qui lui permet un premier ajustage de la largeur de la boucle.

Il insère alors le manchon 35 amovible à frottement doux dans l'orifice 14 de la première branche, en positionnant la partie allongée 40 du manchon 35 dans l'alignement du rétrécissement en vis à vis correspondant dudit orifice de la première branche, la différence de valeur d'angle entre α et γ autorisant un ajustement avec la tige.

Puis il introduit la vis 27 dans l'alésage du manchon jusqu'à venir coopérer avec l'alésage 15 de la seconde branche 7.

Dans un autre mode de réalisation la vis 27 et le manchon 35 sont pré-assemblés et introduits simultanément.

Il y a alors vissage progressif de la vis, ce qui vient pousser le chanfrein 41 contre la tige le long d'une droite génératrice de ladite tige.

La boucle 22 est alors rapprochée de la tige 2, ce qui a pour résultat de rapprocher progressivement la vertèbre 23 de la tige 2 et de redresser la colonne, le frottement des portions d'extrémité contre le fond du U étant dosé.

Dans un autre mode de réalisation correspondant au cas d'une utilisation en dégénératif, il n'y a pas de phase de réduction proprement dite. Il s'agit simplement de mettre la tresse en tension alors que la tige est déjà correctement positionnée. Il n'y a donc qu'un rapprochement minime entre la vertèbre 23 et la tige 2.

Lorsque la position recherchée est obtenue, il y a alors vissage définitif jusqu'à butée longitudinale de l'épaulement 37 de la tête 31 de la vis contre le bord supérieur du manchon 38.

Comme il va de soi et comme il résulte également de ce qui précède, la présente invention n'est pas limitée aux modes de réalisation plus particulièrement décrits. Elle en embrasse au contraire toutes les variantes et notamment celles où deux ou plusieurs tiges sont fixées à la suite ou de part et d'autre de la colonne vertébrale, celle où la vis est de forme différente et/ou le pan coupé n'est pas plan mais assure un contact linéaire avec la tige, c'est-à-dire que le pan est formé par une section bombée autour d'un axe parallèle à l'axe de la tige 2.

Une telle disposition va autoriser un contact sur une surface longitudinale éventuellement plus importante qu'une simple ligne, et de ce fait améliorer encore la pérennité du maintien du lien.

## Revendications

1. Dispositif (1) de fixation vertébrale pour maintien d'une vertèbre (23) rachidienne sur une tige (2), comportant un élément (3) de fixation comprenant une pièce (4) de section transversale en forme de U ou sensiblement en forme de U présentant une première branche (5), un fond (6) et une seconde branche (7), lesdites branches comportant une portion distale, une boucle (22) formée par le rapprochement de deux portions d'extrémité (25', 25") d'une bande (25) souple de liaison de la vertèbre (23) avec la pièce (4), ladite pièce (4) étant munie sur chacune de ses branches (5, 7) d'un évidement (14, 18) de passage des portions d'extrémité (25', 25") de la bande (25), lesdits évidements (14, 18) étant situés en vis à vis l'un de l'autre, et des moyens (26) de blocage réglables des deux portions d'extrémité (25', 25") de la bande (25) souple sur la paroi du fond (6) du U par la tige (2), enfilée sur la pièce (4), comprenant une vis (27) de liaison comportant un corps (18) reliant les portions distales en vis à vis des deux branches du U, ledit corps (18) étant muni d'un côté d'une tête (31) de vissage et de blocage longitudinal de la vis (27) par rapport à une première branche (5) du U et de l'autre côté d'un pas de vis de vissage dans la seconde branche (7) du U, **caractérisé en ce que** les moyens (26) de blocage comportent de plus un manchon (35) amovible de passage de la vis (27) insérable dans la première branche (5) du U, en butée longitudinale avec la tête (31) de la vis (27) et comportant une partie chanfreinée (35") agencée pour coopérer avec la tige (2) et pour comprimer les portions d'extrémité (25', 25") de la bande (25) entre la tige (2) et le fond (6) du U lors du serrage.

2. Dispositif selon la revendication 1, **caractérisé en ce que** la partie chanfreinée (35") est plane et forme un pan coupé (41).

3. Dispositif selon la revendication 2, **caractérisé en ce que** le pan coupé (41) forme un angle (β) compris entre 30° et 80° avec le plan (43) perpendiculaire à l'axe (29) de la vis (27).

4. Dispositif selon la revendication 3, **caractérisé en ce que** le pan coupé (41) forme un angle de 60°.

5. Dispositif selon la revendication 1, **caractérisé en ce que** la partie chanfreinée (35") est concave ou convexe allongée autour d'un axe parallèle ou sensiblement parallèle à la tige (2) enfilée sur la pièce (4).

6. Dispositif selon l'une quelconque des revendications précédentes, **caractérisé en ce que** la pièce (4) est formée d'un seul tenant.

7. Dispositif selon l'une quelconque des revendications précédentes, **caractérisé en ce que** la paroi interne (20) du fond (6) du U est en forme d'une portion de cylindre, terminée d'un côté par un rebord (21) dans le sens parallèle à la tige (2), autorisant un blocage de ladite tige (2) dans le fond (6) du U.

8. Dispositif selon l'une quelconque des revendications précédentes, **caractérisé en ce que** la tête (31) de la vis (27) de serrage comporte un épaulement (37) propre à coopérer avec la face externe (38) de la partie supérieure du manchon (35).

9. Dispositif selon l'une quelconque des revendications précédentes, **caractérisé en ce que** la vis (27), le manchon (35) et la pièce (4) de liaison sont en titane, la tige (2) en polymère, et la bande (25) souple une tresse en polymère.

10. Dispositif selon l'une quelconque des revendications 1 à 8, **caractérisé en ce que** la vis (27) et la pièce (4) de liaison sont en titane, le manchon (35) et la tige (2) sont en polymère et la bande (25) souple est une tresse de polymère.

11. Dispositif selon l'une quelconque des revendications 1 à 8, **caractérisé en ce que** la vis (27) est en titane, le manchon (35) ainsi que la tige (2) et la pièce (4) de liaison sont en polymère et la bande (25) souple est une tresse de polymère.

12. Dispositif selon l'une quelconque des revendications 1 à 8, **caractérisé en ce que** la vis, la pièce (4) de liaison et la tige (2) sont en titane, le manchon (35) est en polymère et la bande (25) souple est une tresse de polymère.

13. Dispositif selon l'une quelconque des revendications précédentes, **caractérisé en ce que**, le manchon (35) comprend une partie supérieure (39) en portion de cylindre (35') d'un côté et se termine de l'autre côté, par une partie allongée plus étroite comportant la partie chanfreinée (35"), la première branche (5) du U comportant un orifice (14) de passage du manchon (35) au moins en partie de forme complémentaire à celle de la partie supérieure (39) dudit manchon (35) et s'étendant vers le fond (6) du U pour former l'évidement (14) de passage des portions extrémité de la bande (25) pour la première branche (5) du U.

14. Dispositif selon la revendication 13, **caractérisé en ce que** la partie allongée (35") plus étroite est biseautée, symétrique par rapport au plan central transversal du manchon et présente deux côtés opposés plans formant entre eux un angle α compris entre 15° et 45°.

15. Dispositif selon la revendication 14, **caractérisé en ce que** l'orifice (14) de passage de la première branche (5) du U s'étend vers le fond (6) pour former l'évidement (14) de passage de la bande (25) en présentant deux côtés latéraux (11, 12) symétriques par rapport à l'axe de la pièce (4) en formant entre eux un angle γ < α.

16. Dispositif selon l'une quelconque des revendications 1 à 13, **caractérisé en ce que** le manchon et la première branche (5) du U comportent des moyens d'indexage de la position du manchon dans ladite première branche (5).

17. Système de redressement d'une colonne vertébrale comprenant au moins deux dispositifs selon l'une quelconque des revendications précédentes, **caractérisé en ce qu'**il comporte au moins une tige (2) cylindrique sur laquelle sont fixés les dispositifs.

18. Méthode de blocage d'une boucle (22) formée par les deux extrémités (25', 25") d'une bande (25) souple sur une tige (2) à l'aide d'une pièce (4) de fixation de section transversale en forme de U, **caractérisée en ce que** on bloque les extrémités (25', 25") entre le fond (6) du U et la tige (2) par vissage et blocage longitudinal d'une vis (27) au travers d'un manchon (35) amovible du passage de la vis ledit manchon étant en butée d'un côté avec une extrémité distale d'une branche du U et de l'autre côté avec la tige (2) via une partie chanfreinée (35").

19. Méthode de blocage selon la revendication 18, **caractérisé en ce que** le manchon (35) pouvant se positionner librement dans un évidement (14) de passage dans une branche du U, on ajuste celui-ci en position avec la tige (2) en fonction de la zone qu'il rencontre en arrivant en contact avec la tige (2).

20. Méthode selon l'une quelconque des revendications 18 et 19, **caractérisé en ce que** la vis (27) et le manchon (35) sont pré-assemblés de façon à être solidaires en ne conservant qu'une liberté de rotation de l'un par rapport à l'autre.

## Patentansprüche

1. Wirbelsäulenfixierungsvorrichtung (1) zum Halten eines Wirbelsäulenwirbels (23) an einem Stift (2), umfassend ein Fixierungselement (3) mit einem Teil (4) mit einem U-förmigen oder im Wesentlichen U-förmigen Querschnitt mit einem ersten Schenkel (5), einem Boden (6) und einem zweiten Schenkel (7), wobei die Schenkel einen distalen Abschnitt aufweisen, eine Öse (22), die durch das Annähern zweier Endabschnitte (25, 25") eines flexiblen Bandes (25) zur Verbindung des Wirbels (23) mit dem Teil (4) gebildet ist, wobei das Teil (4) an seinen beiden Schenkeln (5, 7) mit einer Aussparung (14, 18) für die Durchführung der Endabschnitte (25', 25") des Bandes (25) versehen ist, wobei die Aussparungen (14, 18) einander gegenüberliegen, und Blockierungsmittel (26) der zwei Endabschnitte (25', 25") des flexiblen Bandes (25) an der Bodenwand (6) des U, die durch den auf das Teil (4) aufgefädelten Stift (2) einstellbar sind, umfassend eine Verbindungsschraube (27) mit einem Körper (18), der die distalen, einander gegenüberliegenden Abschnitte der zwei U-Schenkel verbindet, wobei der Körper (18) auf einer Seite mit einem Kopf (31) für die Verschraubung und die Längsblockierung der Schraube (27) relativ zu einem ersten Schenkel (5) des U versehen ist und auf der anderen Seite mit einem Gewindegang für die Verschraubung in dem zweiten Schenkel (7) des U,
**dadurch gekennzeichnet,**
**dass** die Blockierungsmittel (26) zusätzlich eine abnehmbare Hülse (35) für die Durchführung der Schraube (27) aufweisen, die in den ersten Schenkel (5) des U eingeführt werden kann, längsseitig gegen den Kopf (31) der Schraube (27) anschlägt und einen abgeschrägten Bereich (35") aufweist, der angeordnet ist, um mit dem Stift (2) zusammenzuwirken und um die Endabschnitte (25', 25") des Bands (25) zwischen dem Stift (2) und dem Boden (6) des U beim Spannen zusammenzudrücken.

2. Vorrichtung nach Anspruch 1,
**dadurch gekennzeichnet,**
**dass** der abgeschrägte Bereich (35") eben ist und eine abgeschrägte Fläche (41) bildet.

3. Vorrichtung nach Anspruch 2,
**dadurch gekennzeichnet,**
**dass** die abgeschrägte Fläche (41) einen Winkel (β) zwischen 30° und 80° mit der zur Achse (29) der Schraube (27) senkrechten Ebene (43) bildet.

4. Vorrichtung nach Anspruch 3,
**dadurch gekennzeichnet,**
**dass** die abgeschrägte Fläche (41) einen 60°-Winkel bildet.

5. Vorrichtung nach Anspruch 1,
**dadurch gekennzeichnet,**
**dass** der abgeschrägte Bereich (35") konkav oder konvex längs um eine Achse angeordnet ist, die parallel oder im Wesentlichen parallel zu dem auf das Teil (4) aufgefädelten Stift (2) verläuft.

6. Vorrichtung nach einem der vorhergehenden Ansprüche,
**dadurch gekennzeichnet,**
**dass** das Teil (4) einstückig ausgebildet ist.

7. Vorrichtung nach einem der vorhergehenden Ansprüche,
**dadurch gekennzeichnet,**
**dass** die Innenwand (20) des Bodens (6) des U die Form eines Zylinderabschnitts aufweist, der auf einer Seite mit einem Rand (21) parallel zu dem Stift (2) endet, wodurch der Stift (2) am Boden (6) des U blockiert werden kann.

8. Vorrichtung nach einem der vorhergehenden Ansprüche,
**dadurch gekennzeichnet,**
**dass** der Kopf (31) der Klemmschraube (27) einen Absatz (37) aufweist, der geeignet ist, mit der Außenfläche (38) des oberen Teils der Hülse (35) zusammenzuwirken.

9. Vorrichtung nach einem der vorhergehenden Ansprüche,
**dadurch gekennzeichnet,**
**dass** die Schraube (27), die Hülse (35) und das Verbindungsteil (4) aus Titan sind, der Stift (2) aus Polymer besteht und das flexible Band (25) ein Polymergeflecht ist.

10. Vorrichtung nach einem der Ansprüche 1 bis 8,
**dadurch gekennzeichnet,**
**dass** die Schraube (27) und das Verbindungsteil (4) aus Titan sind, die Hülse (35) und der Stift (2) aus Polymer bestehen und das flexible Band (25) ein Polymergeflecht ist.

11. Vorrichtung nach einem der Ansprüche 1 bis 8,
**dadurch gekennzeichnet,**
**dass** die Schraube (27) aus Titan ist, die Hülse (35) sowie der Stift (2) und das Verbindungsteil (4) aus Polymer bestehen und das flexible Band (25) ein Polymergeflecht ist.

12. Vorrichtung nach einem der Ansprüche 1 bis 8,
**dadurch gekennzeichnet,**
**dass** die Schraube, das Verbindungsteil (4) und der Stift (2) aus Titan sind, die Hülse (35) aus Polymer besteht und das flexible Band (25) ein Polymergeflecht ist.

13. Vorrichtung nach einem der vorhergehenden Ansprüche,
**dadurch gekennzeichnet,**
**dass** die Hülse (35) auf einer Seite einen oberen Bereich (39) in Form eines Zylinderabschnitts (35') aufweist und auf der anderen Seite mit einem engeren länglichen Bereich endet, der den abgeschrägten Bereich (35") aufweist, wobei der erste Schenkel (5) des U eine Öffnung (14) für die Durchführung der Hülse (35) aufweist, die zumindest teilweise komplementär zu dem oberen Bereich (39) der Hülse (35) ausgebildet ist und sich zum Boden (6) des U hin erstreckt, um die Aussparung (14) für die Durchführung der Endabschnitte des Bands (25) für den ersten Schenkel (5) des U zu bilden.

14. Vorrichtung nach Anspruch 13,
**dadurch gekennzeichnet,**
**dass** der längliche engere Bereich (35") abgeschrägt ist, symmetrisch zur zentralen Querebene der Hülse verläuft und zwei einander gegenüberliegende ebene Seiten aufweist, die miteinander einen Winkel α zwischen 1° und 45° bilden.

15. Vorrichtung nach Anspruch 14,
**dadurch gekennzeichnet,**
**dass** die Öffnung (14) für die Durchführung des ersten Schenkels (5) des U sich zum Boden (6) hin erstreckt, um die Aussparung (14) für die Durchführung des Bands (25) zu bilden, und dabei zwei Seitenflächen (11, 12) aufweist, die symmetrisch zu der Achse des Teils (4) sind und dabei miteinander einen Winkel γ < α bilden.

16. Vorrichtung nach einem der Ansprüche 1 bis 13,
**dadurch gekennzeichnet,**
**dass** die Hülse und der erste Schenkel (5) des U Mittel für die Kennzeichnung der Position der Hülse in dem ersten Schenkel (5) aufweisen.

17. System zur Aufrichtung einer Wirbelsäule mit mindestens zwei Vorrichtungen nach einem der vorhergehenden Ansprüche,
**dadurch gekennzeichnet,**
**dass** es mindestens einen zylindrischen Stift (2) aufweist, an dem die Vorrichtungen befestigt sind.

18. Verfahren zur Blockierung einer Öse (22), die durch die zwei Enden (25', 25") eines flexiblen Bands (25) an einem Stift (2) mithilfe eines Befestigungsteils (4) mit einem U-förmigen Querschnitt gebildet ist, **dadurch gekennzeichnet, dass** die Enden (25', 25") zwischen dem Boden (6) des U und dem Stift (2) durch Verschraubung und Längsblockierung einer Schraube (27) durch eine abnehmbare Hülse (35) für die Durchführung der Schraube blockiert werden, wobei die Hülse auf einer Seite gegen ein distales Ende eines Schenkels des U und auf der anderen Seite gegen den Stift (2) über einen abgeschrägten Bereich (35") anschlägt.

19. Blockierungsverfahren nach Anspruch 18,
**dadurch gekennzeichnet,**
**dass** bei freier Positionierungsmöglichkeit der Hülse (35) in einer Aussparung (14) für die Durchführung in einem Schenkel des U diese und der Stift (2) positionsmäßig angepasst werden in Abhängigkeit von dem Bereich, auf den sie trifft, wenn sie mit dem Stift (2) in Berührung gelangt.

20. Verfahren nach einem der Ansprüche 18 und 19,
**dadurch gekennzeichnet,**
**dass** die Schraube (27) und die Hülse (35) so vormontiert werden, dass sie fest verbunden sind und dabei lediglich eine Drehbewegungsfreiheit relativ zueinander bewahren.

## Claims

1. Vertebral fixation device (1) for supporting a spinal vertebra (23) on a rod (2), comprising a fixation element (3) comprising a part (4) of transverse section in the shape of a U or substantially in the shape of a U exhibiting a first limb (5), a bottom (6) and a second limb (7), said limbs comprising a distal portion, a loop (22) formed by the rapprochement of two end portions (25', 25") of a flexible band (25) linking the vertebra (23) with the part (4), said part (4) being provided on each of its limbs (5, 7) with a recess (14, 18) for passage of the end portions (25', 25") of the band (25), said recesses (14, 18) being located facing one another, and adjustable means (26) for locking the two end portions (25', 25") of the flexible band (25) on the wall of the bottom (6) of the U by the rod (2), threaded onto the part (4), comprising a linking screw (27) having a body (18) linking the facing distal portions of the two limbs of the U, said body (18) being provided on one side with a head (31) for screwing and longitudinal locking of the screw (27) in relation to a first limb (5) of the U and on the other side a screw thread for screwing in the second limb (7) of the U, **characterised in that** the locking means (26) also comprise a detachable sleeve (35) for passage of the screw (27) which can be inserted in the first limb (5) of the U, in longitudinal abutment with the head (31) of the screw (27) and comprising a chamfered part (35") arranged to co-operate with the rod (2) and to compress the end portions (25', 25") of the band (25) between the rod (2) and the bottom (6) of the U when tightened.

2. Device according to claim 1, **characterised in that** the chamfered part (35") is flat and forms a cut face (41).

3. Device according to claim 2, **characterised in that** the cut face (41) forms an angle (β) of between 30° and 80° with the plane (43) perpendicular to the axis (29) of the screw (27).

4. Device according to claim 3, **characterised in that** the cut face (41) forms an angle of 60°.

5. Device according to claim 1, **characterised in that** the chamfered part (35") is concave or convex and elongated around an axis parallel or substantially parallel to the rod (2) threaded on to the part (4).

6. Device according to any one of the preceding claims, **characterised in that** the part (4) is formed in one piece.

7. Device according to any one of the preceding claims, **characterised in that** the internal wall (20) of the bottom (6) of the U is in the shape of a portion of a cylinder, terminated on one side by an edge (21) in the direction parallel to the rod (2), allowing locking of said rod (2) in the bottom (6) of the U.

8. Device according to any one of the preceding claims, **characterised in that** the head (31) of the clamping screw (27) comprises a shoulder (37) capable of co-operating with the external face (38) of the upper part of the sleeve (35).

9. Device according to any one of the preceding claims, **characterised in that** the screw (27), the sleeve (35) and the linking part (4) are made of titanium, the rod (2) of polymer, and the flexible band (25) of a braid of polymer.

10. Device according to any one of claims 1 to 8, **characterised in that** the screw (27) and the linking part (4) are made of titanium, the sleeve (35) and the rod (2) are made of polymer and the flexible band (25) is a braid of polymer.

11. Device according to any one of claims 1 to 8, **characterised in that** the screw (27) is made of titanium, the sleeve (35) as well as the rod (2) and the linking part (4) are made of polymer and the flexible band (25) is a braid of polymer.

12. Device according to any one of claims 1 to 8, **characterised in that** the screw, the linking part (4) and the rod (2) are made of titanium, the sleeve (35) is made of polymer and the flexible band (25) is a braid of polymer.

13. Device according to any one of the preceding claims, **characterised in that** the sleeve (35) comprises an upper part (39) in the shape of a portion of a cylinder (35') on one side and is terminated on the other side by a narrower elongated part having the chamfered part (35"), the first limb (5) of the U comprising an orifice (14) for passage of the sleeve (35) at least in part in a shape complementing that of the upper part (39) of said sleeve (35) and extending towards the bottom (6) of the U to form the recess (14) for passage of the end portions of the band (25) for the first limb (5) of the U.

14. Device according to claim 13, **characterised in that** the narrower elongated part (35") is bevelled, symmetrical in relation to the transverse central plane of the sleeve and exhibits two opposing sides which are flat and form between them an angle α of between 15° and 45°.

15. Device according to claim 14, **characterised in that** the orifice (14) for passage of the first limb (5) of the U extends towards the bottom (6) to form the recess (14) for passage of the band (25) exhibiting two lateral sides (11, 12) which are symmetrical in relation to the axis of the part (4) forming between them an angle γ < α.

16. Device according to any one of claims 1 to 13, **characterised in that** the sleeve and the first limb (5) of the U comprise means for indexing the position of the sleeve in said first limb (5).

17. System for straightening a vertebral column comprising at least two devices according to any one of the preceding claims, **characterised in that** it comprises at least one cylindrical rod (2) on which the devices are fixed.

18. Method for locking a loop (22) formed by the two ends (25', 25") of a flexible band (25) on a rod (2) with the aid of a fixing part (4) of transverse section in the form of a U, **characterised in that** the ends (25', 25") are locked between the bottom (6) of the U and the rod (2) by screwing and longitudinal locking of a screw (27) through a sleeve (35) which is detachable for passage of the screw, said sleeve being in abutment on one side with a distal end of a limb of the U and on the other side with the rod (2) via a chamfered part (35").

19. Method for locking according to claim 18, **characterised in that**, the sleeve (35) being capable of positioning itself freely in a recess (14) for passage in a limb of the U, the latter is adjusted in position with the rod (2) as a function of the zone that it encounters on coming into contact with the rod (2).

20. Method according to any one of claims 18 and 19, **characterised in that** the screw (27) and the sleeve (35) are preassembled so as to be integral with one another while only allowing freedom of rotation of one in relation to the other.
